(19) Europäisches Patentamt
European Patent Office
Office européen des brevets

(11) **EP 3 549 668 A1**

(12) **EUROPÄISCHE PATENTANMELDUNG**

(43) Veröffentlichungstag:
09.10.2019 Patentblatt 2019/41

(21) Anmeldenummer: 19162076.4

(22) Anmeldetag: 12.03.2019

(51) Int Cl.:
*B01J 21/12* (2006.01)   *B01J 23/02* (2006.01)
*B01J 23/755* (2006.01)   *B01J 35/00* (2006.01)
*B01J 37/00* (2006.01)   *C07C 2/10* (2006.01)

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(30) Priorität: **14.03.2018 EP 18161747**

(71) Anmelder: **Evonik Degussa GmbH**
**45128 Essen (DE)**

(72) Erfinder:
• **Nadolny, Fabian**
**59821 Arnsberg (DE)**

• **Peitz, Stephan**
**45739 Oer-Erkenschwick (DE)**
• **Stochniol, Guido**
**45721 Haltern am See (DE)**
• **Reeker, Helene**
**44227 Dortmund (DE)**
• **Reschetilowski, Wladimir**
**01445 Radebeul (DE)**

(74) Vertreter: **Evonik Patent Association**
**c/o Evonik Industries AG**
**IP Management**
**Bau 1042A/PB 15**
**Paul-Baumann-Straße 1**
**45772 Marl (DE)**

(54) **NI-HALTIGER KATALYSATOR MIT EINEM DEFINIERTEN VERHÄLTNIS VON NI ZU (ERD)ALKALI-IONEN ZUR OLIGOMERISIERUNG VON OLEFINEN**

(57) Gegenstand der Erfindung ist ein Katalysator zur Oligomerisierung von C3-C6 Olefinen, welcher Nickeloxid und Silica-Alumina-Supportmaterial enthält, und ein Verfahren zur Herstellung des Oligomerisierungskatalysators. Der Oligomerisierungskatalysator ist frei von Titan- und Zirkondioxid und umfasst 15 bis 40 Gew.-% Nickeloxid, einen Al-haltigen und Si-freien Binder und ein amorphes Silica-Alumina-Supportmaterial, entsprechend 10-30 Gew.-% Al2O3 und 55 bis 70 Gew.-% SiO2, sowie 0,01 bis 2,5 Gew.-% eines Alkalimetalloxids, mit der Maßgabe, dass das molare Verhältnis der Ionen Ni : (Erd)alkalimetall = 1 : 0,1 bis 1 : 0,001 ist.

EP 3 549 668 A1

**Beschreibung**

Die Erfindung betrifft ein Oligomerisierungskatalysator zur Oligomerisierung von niedermolekularen Olefinen, die Verwendung dieses Katalysators sowie ein Verfahren zur Oligomerisierung von niedermolekularen Olefinen unter Verwendung des erfindungsgemäßen Oligomerisierungskatalysators.

Generell versteht man unter der Oligomerisierung die Reaktion von ungesättigten Kohlenwasserstoffen mit sich selbst, wobei entsprechend längerkettige Kohlenwasserstoffe, die so genannten Oligomere, entstehen. So lässt sich beispielsweise durch die Oligomerisierung von zwei Olefinen mit drei Kohlenstoffatomen ein Olefin mit sechs Kohlenstoffatomen (Hexen) aufbauen. Die Oligomerisierung von zwei Molekülen miteinander wird auch Dimerisierung genannt.

Die erhaltenen Oligomere sind Zwischenprodukte, die beispielsweise für die Herstellung von Aldehyden, Carbonsäuren und Alkoholen eingesetzt werden. Die Oligomerisierung von Olefinen wird großtechnisch entweder in homogener Phase an einem gelösten oder heterogen an einem festen Katalysator oder mit einem zweiphasigen Katalysatorsystem durchgeführt.

Bei den heterogen katalysierten Verfahren ist die Oligomerisierung an sauren Oligomerisierungskatalysatoren lange bekannt. Technisch werden z. B. Zeolithe oder Phosphorsäure auf einem Support eingesetzt. Hierbei werden Isomerengemische von verzweigten Olefinen erhalten. Für die nicht-saure, heterogen katalysierte Oligomerisierung von Olefinen, mit hoher Dimerenselektivität werden in der Technik häufig Nickelverbindungen auf Supportmaterialien eingesetzt. So wird in der WO 95/14647 A1 ein Nickelkatalysator mit einem Supportmaterial, das aus den Komponenten Titanoxid und/oder Zirkoniumoxid, Siliciumoxid und gegebenenfalls Aluminiumoxid besteht, für die Olefinoligomerisierung beschrieben. An diesen Katalysatoren werden Gemische linearer Butene in einer Selektivität von unter 75 % zu C8-Olefinen oligomerisiert.

WO 95/14647 A1 beschreibt ein Verfahren zur Oligomerisierung von Olefinen mittels eines Oligomerisierungskatalysators, der als aktive Bestandteile, nach Abzug des Glühverlustes nach Temperung bei 900°C, 10 bis 70 Gew.-% Nickeloxid, berechnet als NiO, 5 bis 30 Gew.-% Titandioxid und/oder Zirkoniumoxid, 0 bis 20 Gew.-% Aluminiumoxid, 20 bis 40 Gew.-% Siliciumdioxid und 0,01 bis 1 Gew.-% eines Alkalimetalloxids enthält

Es wird vermutet, dass die katalytische Aktivität von nickelbasierten, heterogenen Katalysatoren zur Oligomerisierung von Olefinen, insbesondere Olefinen mit 2 bis 8 Kohlenstoffatomen, auf der Wechselwirkung zwischen Nickel-Kationen und Oberflächen-Aluminiumatomen basiert. Die Zugabe von Titandioxid und/ Zirkoniumdioxid führt jedoch dazu, dass die Gesamtzusammensetzung prozentual weniger Aluminium bzw. Aluminiumoxid enthält, die dazu führt dass die katalystische Aktivität und/oder der Umsatz die mit der als Katalysator eingesetzten Zusammensetzung erreicht werden können, verringert wird. Gleichzeitig kann die Zugabe von Titandioxid und/oder Zirkoniumoxid dazu führen, dass größere Mengen an unerwünschten Oligomerisierungsprodukten, insbesondere stark verzweigten Oligomeren, gebildet werden.

Aufgabe der vorliegenden Erfindung war es, einen verbesserten Oligomerisierungskatalysator bereitzustellen, der die oben genannten Nachteile überwinden kann und der insbesondere zu höheren Selektivitäten zu weniger verzweigten Produkten führt, wobei kein negativer Effekt auf die Standzeit des Katalysators und die mechanischen Eigenschaften wie Festigkeit erfolgen darf.

Es wurde überraschend gefunden, dass der erfindungsgemäße Oligomerisierungskatalysator gemäß Anspruch 1 die Aufgabenstellung erfüllt. Es hat sich überraschend gezeigt, dass durch Verzichten auf Titandioxid und Zirkoniumdioxid und durch Zugabe von vergleichweise geringen Mengen an Alkali- oder Erdalkalikationen ein Oligomerisierungskatalysator bereitgestellt werden kann, welcher bei der Oligomerisierung von Olefinen, insbeosndere Olefinen mit 3 bis 6 Kohlenstoffatomen, eine Steigerung der Selektivität zu linearen Produkten zeigt, ohne dass der Umsatz signifikant verringert wird. Geringe Mengen an Alkali-Kationen im Sinne der vorliegenden Erfindung meint einen mindestens 10-fachen Überschuss von sowohl Nickelionen und optional auch Aluminiumionen im Vergleich zum molaren Anteil der Alkalikationen oder Erdalkalikationen.

Demgemäß ist ein erster Gegenstand der vorliegenden Erfindung ein Oligomerisierungskatalysator, welcher Nickeloxid, Al-haltigen und Si-freien Binder (<0,1 Gew.-% Si), ein Silica-Alumina-Supportmaterial und ein Alkali- oder Erdalkalimetalloxid umfasst, wobei der Katalysator eine Zusammensetzung von 15 bis 40 Gew.-%, vorzugsweise 15 bis 30 Gew.-% NiO, 10 bis 30 Gew.-%, vorzugsweise 12 bis 30 Gew.-% $Al_2O_3$, 55 bis 70 Gew.-% $SiO_2$ und 0,01 bis 2,5 Gew.-%, vorzugsweise 0,05 bis 2 Gew.-% eines Alkalimetall- oder Erdalkalimetalloxids aufweist und wobei der Oligomerisierungskatalysator sich durch ein molares Verhältnis Nickelionen : Alkali-/Erdalkaliionen im Bereich von 1 : 0,1 bis 1 : 0,001 auszeichnet und im Wesentlichen frei von Titandioxid und Zirkoniumdioxid ist.

Als Alkalimetalloxid oder Erdalkalimetalloxid liegt vorzugsweise ein Oxid von Lithium, Natrium, Kalium, Magnesium, Calcium oder eine Mischung daraus, weiterhin bevorzugt von Lithium, Natrium, Kalium oder eine Mischung daraus, besonders bevorzugt von Lithium, Natrium oder eine Mischung daraus, vor. In einer besonders bevorzugten Ausführungsform ist das Alkalimetall- oder Erdalkalimetalloxid ein Lithiumoxid.

Der Binder ist ein Material, das dafür sorgt, dass der erfindungsgemäß hergestellte Katalysator die nötige mechanische Festigkeit aufweist. Mit "namorph" im Sinne der vorliegenden Erfindung ist die Eigenschaft eines Feststoffes gemeint, die daraus folgt, dass der Feststoff keine Kristallstruktur, d. h. keine Fernordnung, aufweist. Im Sinne der vorliegenden Erfindung ist aber nicht auszuschließen, dass das amorphe Silica-Alumina-Supportmaterial kleine kristalline Domänen aufweist. Das amorphe Silica-Alumina-Supportmaterial ist somit kein kristallines Material, beispielsweise kein zeolithisches Material.

Der Begriff im Wesentlichen frei von Titandioxid und/oder Zirkondioxid bedeutet im Sinne der vorliegenden Erfindung, dass der Oligomerisierungsgehalt einen Gehalt an Titandioxid und/oder Zirkondioxid von weniger als 0,5 Gew.-%, vorzugsweise weniger als 0,1 Gew.-%, besonders bevorzugt weniger als 0,01 Gew.-% aufweist.

Erfindungsgemäß bevorzugt weist der Oligomerisierungskatalysator zudem eine spezifische Oberfläche (berechnet nach BET) von 150 bis 400 $m^2/g$, vorzugsweise 190 bis 350 $m^2/g$, besonders bevorzugt von 220 bis 330 $m^2/g$ auf. Die spezifische Oberfläche wird mittels Stickstoff-Physisorption gemäß DIN-ISO 9277 (Stand: 2014-01) gemessen und berechnet.

In einer weiterhin bevorzugten Ausführungsform weist der Oligomerisierungskatalysator Mesoporen und Makroporen auf, hat also eine bimodale Porengrößenverteilung. Die Mesoporen des erfindungsgemäßen Oligomerisierungskatalysators weisen einen mittleren Porendurchmesser von 5 bis 15 nm, vorzugsweise von 7 bis 14 nm, besonders bevorzugt von 9 bis 13 nm auf. Die Makroporen des erfindungsgemäßen Oligomerisierungskatalysators weisen demgegenüber vorzugsweise einen mittleren Porendurchmesser von 1 bis 100 $\mu$m, besonders bevorzugt von 2 bis 50 $\mu$m auf. Das mittlere Porenvolumen des erfindungsgemäßen Oligomerisierungskatalysators, d. h. sowohl der Mesoporen als auch der Makroporen, kann 0,5 bis 1,4 5 $cm^3/g$, vorzugsweise 0,7 bis 1,13 $cm^3/g$ betragen. Der mittlere Porendurchmesser und das mittlere Porenvolumen können mittels Quecksilber-Porosimetrie gemäß DIN 66133 (Stand: 1993-06) bestimmt werden.

Der erfindungsgemäße Oligomerisierungskatalysator liegt vorzugsweise als Granulat vor. Darüber hinaus kann der erfindungsgemäße Oligomerisierungskatalysator einen mittleren Partikeldurchmesser (d50) von 0,1 mm bis 7 mm, vorzugsweise 0,5 bis 6 mm, besonders bevorzugt von 1 mm bis 5 mm aufweisen. Der mittlere Partikeldurchmesser kann mittels bildgebender Verfahren ermittelt werden, insbesondere durch die in den Normen ISO 13322-1 (Stand: 2004-12-01) und ISO 13322-2 (Stand: 2006-11-01) beschriebenen Verfahren. Ein geeignetes Gerät zur Analyse des Partikeldurchmessers ist beispielsweise der Camsizer 2006 (Retsch Technology).

Der Oligomerisierungskatalysator weist in einer weiteren bevorzugten Ausführungsform eine Bulk Crush Strength (BCS) von mehr als 0,5 MPa, vorzugsweise von mehr als 0,6 MPa und besonders bevorzugt von mehr als 0,8 MPa auf. Der BCS-Wert ist ein Maß für die mechanische Festigkeit mineralischer Granulate. Die Bulk Crush Strength (BCS) eines Feststoffes ist ein als Druck in MPa definierter Parameter zu verstehen, bei welchem 0,5 Gew.-% Feinanteil (d.h. Teilchen, die über ein Sieb mit einer Maschenweite von 0,425 mm abgesiebt wurden) gebildet werden, wenn die Feststoffprobe über einen Kolben in einem Rohr mit Druck beaufschlagt wird. Für diesen Zweck werden 20 ml des Feststoffes mit einem Sieb (Maschenweite: 0,425 mm) vorgesiebt, in ein zylindrisches Probenrohr (Innendurchmesser: 27,6 mm, Wandstärke: 5 mm, Höhe: 50 mm) eingefüllt und 5 ml Stahlkugeln (Durchmesser: 3,9 mm) auf die Oberseite des Feststoffes gegeben. Der Feststoff wird anschließend mit unterschiedlichen (zunehmenden) Drücken für drei Minuten beaufschlagt. Anschließend werden die durch die Beaufschlagung mit Druck entstandenen Feinanteile per Siebung abgetrennt, jeweils in Summe gewogen und ihr Prozentanteil bestimmt. Dieses Verfahren wird durchgeführt, bis eine Menge von 0,5 Gew.-% Feinanteil erreicht wird.

Ein Oligomerisierungskatalysator kann auch über seine maximale Schüttdichte charakterisiert werden. In einer bevorzugten Ausführungsform weist der erfindungsgemäße Oligomerisierungskatalysator eine maximale Schüttdichte von 0,1 bis 2 $g/cm^3$, vorzugsweise 0,2 bis 1,5 $g/cm^3$, besonders bevorzugt von 0,3 bis 1,0 $g/cm^3$ auf. Die Bestimmung der Schüttdichte kann über einen Messzylinder erfolgen. In den Messzylinder wird ein bestimmtes Volumen des zu untersuchenden Feststoffes eingefüllt, beispielsweise über eine geeignete Dosiervorrichtung wie den DR100 (Retsch), und der Messzylinder gewogen. Aus dem Gewicht und dem Volumen lässt sich die maximale Schüttdichte ermitteln. Gegebenenfalls muss die Restfeuchte von dem Probengewicht abgezogen werden.

Der erfindungsgemäße Oligomerisierungskatalysator wird durch ein Verfahren hergestellt, welches die folgenden Schritte umfasst:

a) Mischen des amorphen Silica-Alumina-Supportmaterials, des Al-haltigen und Si-freien Binders (<0,1 Gew.-% Si),mindestens eines Teils der Nickelquelle und optional der Alkali- oder Erdalkaliquelle; und Granulieren der so hergestellten Mischung;

a1) Beaufschlagen des in Schritt a) hergestellten Granulats mit mindestens einem Teil einer Nickelquelle und/oder einer einer Alkali- oder Erdalkaliquelle, sofern die gesamte Nickelquelle und/oder die Alkalimetall- oder Erdalkalimetallquelle nicht schon in Schritt a) mit dem amorphen Silica-Alumina-Supportmaterial und dem Al-haltigen und Si-freien Binder vermischt worden ist,
wobei der Anteil des amorphen Silica-Alumina-Supportmaterials an dem Gesamtansatz (Gesamtzusammensetzung inklusive aller ggf. verwendeten Lösemittel) nach Schritt a) oder a1) 20 bis 50 Gew.-%, der Anteil des Al-haltigen und Si-freien Binders an dem Gesamtansatz 5 bis 30 Gew.-%, der Anteil der Alkali- oder Erdalkaliquelle in nicht gelöster Form an dem Gesamtansatz 0,01 und 2,5 Gew.-% und der Anteil der Nickelquelle an dem Gesamtansatz 30 und 50 Gew.-%, beträgt; und

b) Trocknen und Kalzinieren des Granulats zur Herstellung des Oligomerisierungskatalysators.

In einer bevorzugten Ausführungsform werden alle Komponenten (amorphes Silica-Alumina-Supportmaterial, Al-haltiger und Si-freier Binder, Nickelquelle und Alkali- oder Erdalkaliquelle) bereits in Schritt a) vermischt und granuliert. Der Schritt a1) entfällt dabei.

Das in Schritt a) eingesetzte Silica-Alumina-Supportmaterial ist vorzugsweise ein amorphes Alumosilicat. Das amorphe Silica-Alumina-Supportmaterial ist insbesondere kein zeolithisches Material. In einer bevorzugten Ausführungsform ist das Silica-Alumina-Supportmaterial ein amorphes Alumosilicat, welches 10 bis 20 Gew.-%, vorzugsweise 12 bis 17 Gew.-% $Al_2O_3$ und 80 bis 90 Gew.-%, vorzugsweise 83 bis 88 Gew.-% $SiO_2$ umfasst. Weiterhin bevorzugt kann das als das Silica-Alumina-Supportmaterial eingesetzte amorphe Alumosilicat eine Korngröße (d50) im Bereich von 10 bis 80 $\mu$m, vorzugsweise 15 bis 75 $\mu$m aufweisen, gemessen mittels Laserbeugung, beispielsweise einem Mastersizer von Malvern. Darüber hinaus weist das als das Silica-Alumina-Supportmaterial eingesetzte amorphe Alumosilicat vorzugsweise eine spezifische Oberfläche (berechnet nach BET) von 250 bis 380 m$^2$/g, besonders bevorzugt von 280 bis 360 m$^2$/g auf, gemessen mittels Stickstoff-Physisorption gemäß DIN-ISO 9277 (Stand: 2014-01). Der Anteil des Silica-Alumina-Supportmaterials an dem Gesamtansatz (Gesamtzusammensetzung inklusive aller ggf. verwendeten Lösemittel wie Wasser) in Schritt a) beträgt 20 bis 50 Gew.-%, vorzugsweise 25 bis 45 Gew.-%. Wird in Schritt a) nicht die gesamte Nickelquelle zum Gesamtansatz gegeben, sollte eine ausreichende Flüssigkeitsmenge durch Zugabe eines Lösemittels, vorzugsweise Wasser oder eine ammoniakalische Lösung, zur Mischung in Schritt a) hinzugefügt werden, um eine Granulation zu ermöglichen.

Der in Schritt a) ebenfalls eingesetzte Al-haltige und Si-freie Binder (Si-frei bedeutet: <0,1 Gew.-% Si in der Gesamtzusammensetzung des Binders) ist ein oxidisches Aluminiummaterial, vorzugsweise Aluminiumoxid, Aluminiumhydroxid, oder Aluminiumoxid-hydroxid, besonders bevorzugt Boehmit. Der Al-haltige und Si-freie Binder liegt weiterhin bevorzugt nicht in fester Form, sondern in gelöster Form, besonders bevorzugt als kolloidale Lösung vor. Das Lösemittel, indem der Al-haltige und Si-freie Binder, vorzugsweise Aluminiumoxid, Aluminiumhydroxid, oder Aluminiumoxid-hydroxid besonders bevorzugt Boehmit, in gelöster Form, vorzugsweise als kolloidale Lösung vorliegt ist, in einer bevorzugten Ausführungsform eine 1 Gew.-%ige Salpetersäurelösung. Der Al-haltige und Si-freie Binder ist in der kolloidalen Lösung in einer Menge im Bereich von 10 bis 25 Gew.-%, vorzugsweise 12 bis 20 Gew.-%, besonders bevorzugt 14 bis 18 Gew.-% vorhanden. Der Anteil des Al-haltigen und Si-freien Binders an dem Gesamtansatz (Gesamtzusammensetzung inklusive aller ggf. verwendeten Lösemittel wie Wasser) in Schritt a) beträgt 5 bis 30 Gew.-%, vorzugsweise 7 bis 25 Gew.-%.

Zur Mischung in Schritt a) oder zum Granulat in Schritt a1) wird zudem eine Alkali- oder Erdalkaliquelle, vorzugsweise eine Alkalimetall- oder Erdalkalimetallverbindung gegeben. Die Alkalimetall- oder Erdalkalimetallverbindung kann insbesondere eine Lithiumverbindung, Natriumverbindung, Kaliumverbindung, Magnesiumverbindung, Calciumverbindung oder eine Mischung daraus, vorzugsweise eine Lithiumverbindung, Natriumverbindung, Kaliumverbindung oder eine Mischung daraus, besonders bevorzugt eine Lithiumverbindung, eine Natriumverbindung oder eine Mischung daraus sein. In einer besonders bevorzugten Ausführungsform ist die Alkalimetall- oder Erdalkalimetallverbindung eine Lithiumverbindung. Als Alkalimetall- oder Erdalkalimetallverbindung können insbesondere Alkalimetall- oder Erdalkalimetallsalze, vorzugsweise wasserlösliche Alkalimetall- oder Erdalkalimetallsalze, der genannten Verbindungen eingesetzt werden.

Bevorzugte Alkali- oder Erdalkalisalze sind Nitrate, Carbonate oder Hydrogencarbonate von Lithium, Natrium, Kalium, Magnesium oder Calcium. Als Alkalimetall- oder Erdalkalimetallverbindung können insbesondere Natriumcarbonat, Natriumhydrogencarbonat, Kaliumcarbonat, Kaliumhydrogencarbonat, Lithiumcarbonat oder Lithiumhydrogencarbonat verwendet werden. In einer besonders bevorzugten Ausführungsform wird die Alkali- oder Erdalkaliquelle, vorzugsweise die Alkalimetall- oder Erdalkalimetallverbindung als wässrige Lösung hinzugegeben. In einer weiterhin bevorzugten Ausführungsform wird die Alkali- oder Erdalkaliquelle in einer Lösung mit der Nickelverbindung zur Mischung in Schritt a) und/oder zum Granulat in Schritt a1) hinzugefügt. Der Anteil der Alkali- oder Erdalkaliquelle (in nicht gelöster Form) an dem Gesamtansatz (Gesamtzusammensetzung aller ggf. verwendeten Lösemittel wie Wasser) in Schritt a) oder a1) des Herstellverfahrens beträgt zwischen 0,01 und 2,5 Gew.-%, vorzugsweise 0,05 und 2 Gew.-%.

Als Nickelquelle in Schritt a) und/oder a1) kann prinzipiell jede lösliche Nickelverbindung eingesetzt werden. Darunter fallen Nickelnitrat ($Ni(NO_3)_2$), Nickelacetat ($Ni(ac)_2$), Nickelacetylacetonat ($Ni(acac)_2$), Nickelsulfat ($NiSO_4$), Nickelcitrat oder Nickelcarbonat ($NiCO_3$). Bevorzugt sind Nickelnitrat ($Ni(NO_3)_2$), Nickelsulfat ($NiSO_4$), Nickelcarbonat ($NiCO_3$). Als Nickelquelle können Lösungen der vorgenannten Nickelverbindungen, Pasten aus den vorgenannten Nickelverbindungen oder eine Kombination aus Nickel-Lösung und Nickel-Paste eingesetzt werden.

Die Nickel-Lösung ist vorzugsweise eine wässrige oder ammoniakalische Lösung. Eine ammoniakalische Lösung ist eine wässrige Lösung, welche mit Ammoniak versetzt wurde. Die Nickel-Paste enthält vorzugsweise Wasser, wobei die Nickel-Paste gemäß der vorliegenden Erfindung weniger Wasser enthält als die Nickel-Lösung (wenn man von der gleichen Menge an Nickelverbindung ausgeht). Wird die die Alkali- oder Erdalkaliquelle in einer Lösung mit der Nickelverbindung zur Mischung hinzugefügt, ist vorzugsweise ein molares Verhältnis von Nickel zu Alkali- oder Erdalkali-Kationen von 1 : 0,001 bis 1 : 0,1 einzustellen.

Die Nickel-Paste ist prinzipiell ein angefeuchteter Feststoff aus einer Nickelverbindung, die unvollständig hydratisiert ist und bei der sich formal auch hydroxidische Nickelverbindung bilden, bei Nickelcarbonat beispielsweise $NiCO_3*Ni(OH)_2$, aber auch nicht-stöchiometrische Nickelcarbonat-Hydroxide. Die Nickel-Paste enthält in einer bevorzugten Ausführungsform zwischen 30 und 50 Gew.-%, vorzugsweise 35 bis 45 Gew.-% Nickel. bezogen auf das Gesamtgewicht der Paste. Die Nickellösung kann Nickel in einer Menge im Bereich von 1 bis 20 Gew.-%, vorzugsweise 5 bis 15 Gew.-%, enthalten, jeweils bezogen auf das Gesamtgewicht der Lösung.

In einer bevorzugten Ausführungsform wird als Nickel-Lösung eine wässrige, ammoniakalische $Ni(CO_3)$-Lösung, genannt NiHAC-Lösung (es bildet sich in der Lösung ein Nickelhexamincarbonat-Komplex ($[Ni(NH_3)_6]CO_3$)), verwendet, die einen Nickelgehalt im Bereich von 1 bis 20 Gew.-%, vorzugsweise 5 bis 15 Gew.-% aufweist. Als Nickel-Paste wird eine Paste aus Nickelcarbonat und Wasser als Lösemittel verwendet, wobei das Nickel als Carbonat/Hydroxid vorliegt (allgemeine Summenformel $NiCO_3*Ni(OH)_2$), es können aber auch nicht-stöchiometrische Nickelcarbonat-Hydroxide gebildet werden). Die Paste kann einen Nickelgehalt im Bereich von 30 und 50 Gew.-%, vorzugsweise 35 bis 45 Gew.-% aufweisen.

In einer besonders bevorzugten Ausführungsform werden bei der Herstellung des Oligomerisierungskatalysators in Schritt a) und/oder optional a1) sowohl eine NiHAC-Lösung als auch eine Nickelcarbonat-Paste verwendet. Das ist so zu verstehen, dass wenn die Zugabe der Nickelquelle ausschließlich im vorgenannten Schritt a) erfolgt, die Nickelquelle sowohl in Form einer Paste als auch in Form einer Lösung zugegeben werden kann. Andererseits ist das so zu verstehen, dass wenn die Zugabe der Nickelquelle teilweise in Schritt a) und teilweise in Schritt a1) erfolgt, die Nickelquelle in dem einen Schritt a) oder a1) in Form einer Paste und im anderen Schritt a) oder a1) in Form einer Lösung zugegeben werden kann oder in beiden Schritten a) oder a1) sowohl in Form einer Paste als auch in Form einer Lösung zugegeben werden kann. In einer besonders bevorzugten Ausführungsform wird zur Mischung in Schritt a) ist der zumindest eine Teil der Nickelquelle, der zur Mischung hinzugefügt wird, eine Nickel-Paste.

Der Anteil der Nickelquelle (Paste und/oder Lösung) an dem Gesamtansatz (Gesamtzusammensetzung aller ggf. verwendeten Lösemittel wie Wasser) in Schritt a) und optional a1) des Herstellverfahrens beträgt 30 und 50 Gew.-%, vorzugsweise 35 und 45 Gew.-%.

Das erfindungsgemäße Verfahren zeichnet sich insbesondere dadurch aus, dass in Schritt a) oder a1) kein Titandioxid und kein Zirkoniumdioxid zur Mischung gegeben wird, sondern der Oligomerisierungskatalysator ohne Hinzufügen von Titandioxid und Zirkoniumdioxid hergestellt wird. Mögliche Vorkommen von Titandioxid und/oder Zirkoniumdioxid in der Gesamtzusammensetzung des Oligomerisierungskatalysators stammen aus Verunreinigungen bzw. Spurenvorkommen in den eingesetzten Komponenten.

Im Schritt a) werden die einzelnen Komponenten, d. h. das Silica-Alumina-Supportmaterial, der Al-haltige und Si-freie Binder und optional die Nickelquelle, miteinander in einem Mischbehälter unter Verwendung eines Wirblers vermischt und gleichzeitig oder anschließend granuliert. Dazu kann beispielsweise ein Intensivmischer verwendet werden. Die Vermischung und die Granulation können typischerweise bei Umgebungsdruck durchgeführt werden. Die Temperatur, bei der Vermischung und Granulation stattfinden können, liegt vorzugsweise im Bereich von 10 bis 60 °C. Die Dauer des Verfahrensschritts a), also der Vermischung und der Granulation, beträgt zwischen 5 Minuten und 1 Stunde, vorzugsweise zwischen 10 und 30 Minuten.

Im optionalen Schritt a1) wird der restliche Teil der Nickelquelle, vorzugsweise in Form einer Paste oder einer Lösung, zu dem in Schritt a) hergestellten Granulat gegeben und mit dem Granulat vermischt, um das Granulat mit Nickel zu beaufschlagen. Sollte zumindest ein Teil der Nickelquelle in Schritt a1) zugegeben werden, kann das ggf. feuchte Granulat aus Schritt a) vor dem Beaufschlagen mit der Nickelquelle getrocknet werden. Die Trocknungstemperatur kann 80 bis 250 °C, vorzugsweise 100 bis 220 °C betragen.

Das aus Schritt a) und/oder Schritt a1) resultierende Granulat kann noch mindestens einen Teil der verwendeten Lösemittel, insbesondere Wasser, enthalten. Es kann sich also um ein feuchtes Granulat handeln. Bevor das gegebenenfalls noch feuchte Granulat der Kalzination in Schritt b) unterworfen wird, kann das feuchte Granulat gesiebt werden, vorzugsweise mit einem Sieb mit einer Maschenweite von 0,1 bis 1,5 mm. Der abgesiebte Teil des Granulats (Unterkorn) kann zurück zu Schritt a) der Granulation zurückgeführt werden.

Nach dem Vermischen und Granulieren in Schritt a), optional nach dem Beaufschlagen (Imprägnieren) des Granulats mit mindestens einem Teil einer Nickelquelle in Schritt a1) und optional nach dem Sieben des feuchten Granulats kann das Granulat in Schritt b) zunächst getrocknet werden. Dazu können bekannte Geräte wie beispielsweise Bandtrockner oder ähnliches verwendet werden. Die Trocknungstemperatur kann im Bereich von 80 bis 250 °C, vorzugsweise im Bereich von 100 bis 220 °C liegen.

Bevor das optional getrocknete Granulat der Kalzination unterworfen wird, kann das getrocknete Granulat fraktioniert werden, um eine bestimmte Korngröße des Granulats einzustellen. Eine solche Fraktionierung kann beispielsweise durch die Verwendung mindestens eines Siebs mit einer definierten Maschenweite erreicht werden. In einer besonders bevorzugten Ausführungsform werden zwei Siebe verwendet, wobei das eine Sieb eine Maschenweite von 0,1 bis 1,5 mm und das andere Sieb eine Maschenweite von 2,5 bis 7 mm aufweist. Die übrigen Fraktionen (Ober- und Unterkorn) können ggf. nach vorheriger Mahlung zum Schritt a) zurückgeführt werden.

Nach der optionalen Trocknung und eventuellen Fraktionierung des Granulats erfolgt die Kalzination des Granulats. Dabei kann das Granulat in einem geeigneten Ofen, vorzugsweise im Stickstoffstrom, besonders bevorzugt Stickstoffgegenstrom, erhitzt werden. Dem Stickstoffstrom kann während der Kalzination Luft hinzugefügt werden, wobei die Menge der zugeführten Luft 100 bis 10000 Vol.-ppm, vorzugsweise 300 bis 7000 Vol.-ppm betragen kann. Die Kalzinationstemperatur kann 400 bis 800 °C, vorzugsweise 450 bis 700 °C, besonders bevorzugt 500 bis 600 °C betragen. Diese Temperatur kann über mehrere Stunden, vorzugsweise 5 bis 20 Stunde, besonders bevorzugt 8 bis 15 Stunden gehalten werden, bevor das Granulat abgekühlt wird. Beim Abkühlen kann in den Ofen Luft eingeleitet werden, die Menge der eingeleiteten Luft sollte aber kontrolliert werden. Die Menge der optional zugeführten Luft beträgt 100 bis 10000 ppm, vorzugsweise 300 bis 7000 ppm .

Danach kann das abgekühlte Granulat bzw. der fertige Oligomerisierungskatalysator, möglicherweise noch einmal fraktioniert werden, um eine bestimmte Korngröße des abgekühlten Granulats einzustellen. Eine solche Fraktionierung kann beispielsweise durch die Verwendung mindestens eines Siebs mit einer definierten Maschenweite erreicht werden. In einer besonders bevorzugten Ausführungsform werden zwei Siebe verwendet, wobei das eine Sieb eine Maschenweite von 0,1 bis 1,5 mm und das andere Sieb eine Maschenweite von 2,5 bis 7 mm aufweist. Die übrigen Fraktionen (Ober- und Unterkorn) können ggf. nach vorheriger Mahlung zum Schritt a) zurückgeführt werden.

Der so hergestellte Oligomerisierungskatalysator weist nach dem letzten Verfahrensschritt, der Kalzination bzw. einer nachgeschalteten Fraktionierung nach Abkühlung, eine finale Gesamtzusammensetzung von 15 bis 40 Gew.-%, vorzugsweise 15 bis 30 Gew.-% NiO, 10 bis 30 Gew.-%, vorzugsweise 12 bis 30 Gew.-% $Al_2O_3$, 55 bis 70 Gew.-% $SiO_2$ und 0,01 bis 2,5 Gew.-%, vorzugsweise 0,05 bis 2 Gew.-% eines Alkali- oder Erdalkalimetalloxids auf. Die Angaben beziehen sich auf eine Gesamtzusammensetzung von 100 Gew.-%.

Mit steigender Einsatzzeit des Oligomerisierungskatalysators bei der Oligomerisierung kann es zu einer Abnahme des Umsatzes und/oder der Selektivität kommen. Der erfindungsgemäße Katalysator kann nach dem Einsatz in der Oligomerisierungsreaktion regeneriert werden.

Die Regenerierung des Oligomerisierungskatalysators im gebrauchten Zustand umfasst die Schritte:

c) Abbrand; und

d) Wiederherstellung der aktiven Oberflächenstruktur des Oligomerisierungskatalysators.

Es ist möglich, dass der Oligomerisierungskatalysator Zustandnach seinem Einsatz in Oligomerisierungsreaktionen Ablagerungen organischer Stoffe aufweist, die entfernt werden müssen. Die Entfernung der im Katalysator abgelagerten organischen Verbindungen geschieht vorzugsweise in Schritt c) durch Abbrand (Oxidation), wodurch Kohlenstoffoxide und Wasser entstehen. Der Abbrandschritt c) kann in einem Ofen, beispielweise in einem Drehrohrofen oder einem Schachtofen kontinuierlich oder diskontinuierlich durchgeführt werden. Dazu wird der Oligomerisierungskatalysators (in Form eines Granulats) dem Ofen zugeführt und vorzugsweise bei einer vorgegebenen Ofentemperatur von 400 bis 600 °C, besonders bevorzugt von 500 bis 600 °C gehalten. Die beim Abbrand verwendete Verbrennungsluft wird im Gegenstrom zugeführt, optional wird zusätzlich weitere Luft über geeignete Einlässe in das Granulat (Oligomerisierungskatalysator) eingeblasen, um einen schnellen Abbrand zu gewährleisten.

Schritt d), also die Wiederherstellung der aktiven Oberflächenstruktur des Oligomerisierungskatalysators kann in einem Schritt d1) eine (zusätzliche) Beaufschlagung (Imprägnierung) mit Nickel umfassen. Die Beaufschlagung mit Nickel kann analog zur Herstellung des Oligomerisierungskatalysators (Schritt a1)) erfolgen, optional jedoch mit dem Unterschied, dass eine Nickel-Lösung mit einer geringeren NickelverbindungenNickelkonzentration als bei der Herstellung des Oligomerisierungskatalysators verwendet werden kann. Eine Nickel-Paste wird üblicherweise nicht für die Regenerierung eingesetzt. Dabei kommt es darauf an, zusätzliche Mengen an Nickel auf dem Oligomerisierungskatalysator abzuscheiden. Prinzipiell kann dazu jede lösliche Nickelverbindung wie Nickelnitrat ($Ni(NO_3)_2$), Nickelacetat ($Ni(ac)_2$), Nickelacetylacetonat ($Ni(acac)_2$), Nickelsulfat ($NiSO_4$) oder Nickelcarbonat ($NiCO_3$) zur Herstellung einer wässrigen oder ammoniakalischen Nickel-Lösung verwendet werden.

Als besonders vorteilhaft hat sich die Verwendung von NiHAC-Lösungen erwiesen, die durch Auflösen von Nickelcarbonat ($NiCO_3$) in konzentrierten Ammoniaklösungen, ggf. mit Zusatz von Ammoniumcarbonat erhältlich sind. Derartige Lösungen können mit Nickelgehalten von 0,5 bis 14 Gew.-%, insbesondere von 2 bis 10 Gew.-%, ganz besonders von 4 bis 8 Gew.-% für die Imprägnierung verwendet werden.

Zum Nickelauftrag wird der in Schritt c) abgebrannte Oligomerisierungskatalysator beispielsweise mit einer NiHAC-Lösung mit Nickelgehalten von 0,5 bis 14 Gew.-%, insbesondere von 2 bis 10 Gew.-%, ganz besonders von 4 bis 8 Gew.-% bis zur Sättigung der Poren imprägniert. Die Imprägnierung kann mit einem dem Fachmann geläufigen Verfahren wie beispielsweise durch Besprühen bis zum permanenten Auftreten eines Flüssigkeitsfilmes auf der Oberfläche (incipient wetness) durchgeführt werden. Beträgt die Lösungsaufnahme etwa 0,8 bis 1,2 g Lösung pro g Oligomerisierungskatalysator, kann man erreichen, dass etwa 0,5 bis 6 Gew.-% an zusätzlichem Nickel in Form eines basischen Carbonates abgeschieden werden.

Wird der Oligomerisierungskatalysator einem Schritt d1) unterworfen, d.h. mit Nickel beaufschlagt, sollte der Oligomerisierungskatalysator in einer geeigneten Trocknungsapparatur, beispielsweise einem Bandtrockner mit Luftstrom oder auch einem Konustrockner, bei Temperaturen zwischen 100 und 250 °C, vorzugsweise zwischen 120 und 220 °C und Normaldruck oder auch im Vakuum getrocknet werden.

Schritt d) umfasst mindestens den Schritt d2), die Kalzination, die nach einem optionalen Schritt d1) durchgeführt werden würde. Die Kalzination des Oligomerisierungskatalysators kann in einem geeigneten Ofen, wie beispielsweise einem Schachtofen oder Drehrohrofen, kontinuierlich oder diskontinuierlich durchgeführt werden. Bei einer kontinuierlichen Kalzination in Schritt d2) wird weiterhin bevorzugt ein Gas im Gegenstrom durch den Oligomerisierungskatalysator (Granulat) geleitet. Als Gas kann Luft, Stickstoff oder eine Mischung davon verwendet werden. Der Gasstrom kann 0,2 bis 4 m$^3$ Gas pro kg Granulat und Stunde und die Eintrittstemperatur des Gases von 400 bis 800 °C, vorzugsweise 450 bis 700 °C betragen. Zusätzlich zu dieser über das Gas eingetragenen Wärme kann Energie durch aktives Heizen der Wände des Ofens eingetragen werden.

Die Kalzinationstemperatur in dem Ofen kann 400 bis 800 °C, vorzugsweise 450 bis 700 °C, besonders bevorzugt 500 bis 600 °C betragen. Diese Temperatur kann über mehrere Stunden, vorzugsweise 5 bis 60 Stunden, besonders bevorzugt 10 bis 40 Stunden gehalten werden, bevor das Granulat abgekühlt wird. Das Abkühlen findet vorzugsweise im Stickstoffstrom statt. Dem Stickstoff kann zusätzlich Luft hinzugefügt werden, wobei die Luftmenge vorzugsweise kontrolliert werden sollte. Die Menge an Luft, die dem Stickstoff vorzugsweise hinzugefügt wird, kann 100 bis 10000 Vol.-ppm, bevorzugt 300 bis 7000 Vol.-ppm betragen.

Der erfindungsgemäße Oligomerisierungskatalysator bzw. der mit dem erfindungsgemäßen Verfahren hergestellte oder regenerierte Katalysator kann insbesondere für die Oligomerisierung von C3- bis C6-Olefinen, vorzugsweise C3- bis C5-Olefinen, besonders bevorzugt C4-Olefinen oder darauf basierenden olefinhaltigen Einsatzgemischen verwendet werden. Die Olefine oder olefinhaltigen Einsatzgemische werden als Eduktstrom eingesetzt.

Gegenstand der vorliegenden Erfindung ist somit auch ein Verfahren zur Oligomerisierung von C3- bis C6-Olefinen, wobei ein olefinhaltiges Einsatzgemisch, welches die C3- bis C6-Olefine enthält, in mindestens einer Reaktionszone über einen Katalysator geleitet wird, wobei der erfindungsgemäße Oligomerisierungskatalysator zur Katalyse der Oligomerisierungsreaktion eingesetzt wird. Eine Reaktionszone umfasst erfindungsgemäß mindestens einen Reaktor und mindestens ein Destillationskolonne, in der die gebildeten Oligomere abgetrennt werden können. Das erfindungsgemäße Verfahren kann auch mit zwei oder mehr Reaktionszonen betrieben werden. Die Oligomerisierung findet vorzugsweise in flüssiger Phase statt.

Als Olefine für das erfindungsgemäße Verfahren werden C3- bis C6-Olefine, bevorzugt C3- bis C5-Olefine, besonders bevorzugt C4-Olefine oder darauf basierende olefinhaltige Einsatzgemische, die auch Anteile an analogen Alkanen enthalten können, eingesetzt. Geeignete Olefine sind unter anderem α-Olefine, n-Olefine und Cycloalkene. Bevorzugt sind die als Edukte eingesetzten n-Olefine. In einer besonders bevorzugten Ausführungsform handelt es sich bei dem Olefin um n-Buten. Der Begriff 'darauf basierende olefinhaltige Einsatzgemische' ist erfindungsgemäß so zu verstehen, dass er jegliche Art von Gemischen betrifft, die die entsprechenden zu oligomerisierenden C3- bis C6-Olefine in einer Menge enthalten, die es ermöglicht, die Oligomerisierung durchzuführen. Bevorzugt enthalten die olefinhaltigen Einsatzgemische praktisch keine weiteren ungesättigten Verbindungen und mehrfach ungesättigte Verbindungen wie Diene oder Acetylenderivate. Vorzugsweise werden olefinhaltige Einsatzgemische eingesetzt, die weniger als 5 Gew.-%, insbesondere weniger als 2 Gew.-% an verzweigten Olefinen bezogen auf den Olefinanteil enthalten. Weiterhin bevorzugt werden olefinhaltige Einsatzgemische eingesetzt, die weniger als 2 Gew.-% verzweigte Olefine, insbesondere iso-Olefine, wie Isobuten, enthalten.

Propylen (C3) wird großtechnisch durch Spaltung von Naphtha hergestellt und ist eine Grundchemikalie, die leicht verfügbar ist. C5-Olefine sind in Leichtbenzinfraktionen aus Raffinerien oder Crackern enthalten. Technische Gemische, die lineare C4-Olefine enthalten, sind Leichtbenzinfraktionen aus Raffinerien, C4-Fraktionen aus FC- oder Steamcrackern, Gemische aus Fischer-Tropsch-Synthesen, Gemische aus Dehydrierung von Butanen und durch Metathese oder aus anderen technischen Prozessen entstandene Gemische. Beispielsweise können für das erfindungsgemäße Verfahren geeignete Gemische linearer Butene aus der C4-Fraktion eines Steamcrackers gewonnen werden. Dabei wird im ersten Schritt Butadien entfernt. Dies geschieht entweder durch Extraktion oder Extraktionsdestillation des Butadiens oder dessen Selektivhydrierung. In beiden Fällen wird ein praktisch butadienfreier C4-Schnitt erhalten, das Raffinat I. Im zweiten Schritt wird Isobuten aus dem C4-Strom entfernt, z. B. durch Herstellung von Methyl-tert.-butylether (MTBE) durch Umsetzung mit Methanol. Andere Möglichkeiten sind die Umsetzung des Isobutens aus dem Raffinat I mit Wasser zu tert.-Butanol oder die sauer katalysierte Oligomerisierung des Isobutens zu Diisobuten. Der jetzt isobutenfreie C4-Schnitt, das Raffinat II, enthält wie gewünscht die linearen Butene und gegebenenfalls Butane. Optional kann noch das 1-Buten destillativ abgetrennt werden. Beide Fraktionen, die mit But-1-en oder die mit But-2-en, können im erfindungsgemäßen Verfahren eingesetzt werden.

In einer weiteren bevorzugten Ausführungsform werden C4-olefinhaltige Stoffströme dem Verfahren als olefinhaltige Einsatzgemische zugeführt. Geeignete olefinhaltige Einsatzgemische sind unter anderem das Raffinat I (butadienfreier C4-Schnitt des Steamcrackers) sowie das Raffinat II (butadien- und isobutenfreier C4-Schnitt des Steamcrackers).

Eine weitere Möglichkeit, geeignete olefinhaltige Einsatzgemische herzustellen, besteht darin, Raffinat I, Raffinat II oder ein ähnlich zusammengesetztes Kohlenwasserstoffgemisch in einer Reaktivkolonne zu hydroisomerisieren. Dabei kann u. a. ein Gemisch gewonnen werden, das aus 2-Butenen, geringen Anteilen 1-Buten und gegebenenfalls n-Butan sowie Isobutan und Isobuten besteht.

Die Oligomerisierung erfolgt in der Regel bei einer Temperatur im Bereich von 50 bis 200 °C, vorzugsweise 60 bis 180 °C, bevorzugt im Bereich von 60 bis 130°C, und bei einem Druck von 10 bis 70 bar, bevorzugt von 20 bis 55 bar. Sofern die Oligomerisierung in flüssiger Phase erfolgen soll, müssen die Parameter Druck und Temperatur hierfür so gewählt werden, dass der Eduktstrom (die eingesetzten Olefine oder olefinhaltigen Einsatzgemische) in flüssiger Phase vorliegt. Die gewichtbasierten Raumgeschwindigkeiten (Reaktandmasse pro Katalysatormasse pro Zeit; weight hourly space velocity (WHSV)) befinden sich im Bereich zwischen 1 g Reaktand pro g Katalysator und pro h (= 1 $h^{-1}$) und 190 $h^{-1}$, vorzugsweise zwischen 2 $h^{-1}$ und 35 $h^{-1}$, besonders bevorzugt zwischen 3 $h^{-1}$ und 25 $h^{-1}$.

In einer Ausführungsform beträgt der Grad der Dimerisierung (auch "prozentuale Selektivität bezogen auf die Dimerisierung" genannt) nach der Oligomerisierung bezogen auf das umgesetzte Edukt mindestens 60 %, weiter bevorzugt mindestens 75 %, besonders bevorzugt mindestens 80%.

Die Linearität eines Oligomerisierungsprodukts bzw. von den entstandenen Dimeren wird durch den ISO-Index beschrieben und stellt einen Wert für die mittlere Anzahl an Methylverzweigungen im Dimer dar. So tragen (für Buten als Edukt) z.B. n-Oktene mit 0, Methylheptene mit 1 und Dimethylhexene mit 2 zum ISO-Index einer C8-Fraktion bei. Je niedriger der ISO-Index ist, umso linearer sind die Moleküle in der jeweiligen Fraktion aufgebaut. Der ISO-Index errechnet sich nach folgender allgemeiner Formel:

$$\frac{\text{(einfach verzweigte Dimere (Gew.-\%) + 2 x zweifach verzweigte Dimere (Gew.-\%))}}{100}$$

Demnach besitzt ein Dimerengemisch mit einem ISO-Index von 1,0 im Mittel genau eine Methylverzweigung pro Dimerenmolekül.

Der ISO-Index des Produkts aus dem erfindungsgemäßen Oligomerisierungsverfahren beträgt vorzugsweise 0,8 bis 1,2, besonders bevorzugt 0,8 bis 1,1.

Die nach dem erfindungsgemäßen Verfahren hergestellten Oligomere werden unter anderem zur Herstellung von Aldehyden, Alkoholen und Carbonsäuren genutzt. So ergibt beispielsweise das Dimerisat aus linearen Butenen durch Hydroformylierung ein Nonanalgemisch. Dieses liefert entweder durch Oxidation die entsprechenden Carbonsäuren oder durch Hydrierung ein C9-Alkoholgemisch. Das C9-Säuregemisch kann zur Herstellung von Schmiermitteln oder Sikkative verwendet werden. Das C9-Alkoholgemisch ist Vorstufe für die Herstellung von Weichmachern, insbesondere von Di-Nonyl-phthalaten oder DINCH.

Auch ohne weitere Ausführungen wird davon ausgegangen, dass ein Fachmann die obige Beschreibung im weitesten Umfang nutzen kann. Die bevorzugten Ausführungsformen und Beispiele sind deswegen lediglich als beschreibende, keinesfalls als in irgendeiner Weise limitierende Offenbarung aufzufassen.

Nachfolgend wird die vorliegende Erfindung anhand von Beispielen näher erläutert. Alternative Ausführungsformen der vorliegenden Erfindung sind in analoger Weise erhältlich.

Beispiele:

Herstellung des Katalysators 1a (Zugabe von Natrium):

In den Mischbehälter eines Intensivmischers werden ein Binder (Lösung aus Boehmit und einer 1-Gew.-% Salpetersäurelösung, Aluminium-Gehalt zwischen 15 bis 17 Gew.-%), eine Nickelquelle (Nickelpaste, angefeuchtetes Nickelcarbonat, Nickelgehalt zwischen 40 bis 42 Gew.-%), und amorphes Silica-Alumina (77,2 Gew.-% $SiO_2$, 12,2 Gew.-% $Al_2O_3$, Rest: Wasser, Ammoniak, Spuren weiterer Oxide, mittlere Partikelgröße von 22 μm, spezifische Oberfläche von 320 m2/g) gegeben.

Silica-Alumina, Binder und feste Nickelquelle werden in dem Intensivmischer vermischt. Während der Durchmischung werden zusätzlich als flüssige Komponenten eine NiHAC-Lösung (Nickelcarbonat gelöst in konzentrierter ammoniakalischer Lösung, Nickelgehalt zwischen 11 und 12,5%) und eine Alkalimetallverbindung (Natriumcarbonat gelöst in destilliertem Wasser, Verhältnis von Nickel : Natrium etwa 1 : 0,07) über einen Trichter langsam in den Mischbehälter gegeben.

Nachdem alle Komponenten zugegeben wurden, wird das Gemisch für eine effektive Verteilung bei relativ geringer Drehzahl gerührt. Durch eine darauffolgende Erhöhung der Drehzahl des Rührers setzt langsam eine Verdichtung und Granulierung der Masse ein. Die Rührung wird gestoppt, sobald Granulate mit einem geeigneten Partikeldurchmesser (0,1 mm bis 7 mm) erhalten werden. Das so erhaltene Granulat wird bei etwa 120 °C getrocknet und danach mithilfe von zwei Sieben gesiebt, um zu kleine oder zu große Partikel aus dem Granulat zu entfernen.

Anschließend wird Granulat in einem Ofen kalziniert. Für die Kalzination wird das Granulat auf eine Temperatur zwischen 500 bis 600 °C geheizt und diese Temperatur für etwa 10 bis 12 Stunden gehalten. Der mit Granulat gefüllte Ofen wird mit Stickstoff durchströmt, wobei ein Verhältnis von Volumen an Granulat zu Volumen an Stickstoff pro Stunde (Normvolumen) von mindestens 1:1000 eingehalten wird. Während der Abkühlung des Granulats auf Raumtemperatur werden ca. 1000 bis 10000 Vol.-ppm Luft in den Stickstoffstrom dosiert. Das abgekühlte Granulat entspricht dem fertigen Oligomerisierungskatalysator.

Herstellung des Katalysators 1b (Zugabe von Lithium):

Der Katalysator 1b wurde analog zum für den Katalysator 1a beschriebenen Verfahren hergestellt, mit dem Unterschied, dass dabei kein Natriumcarbonat, sondern eine zum für den Katalysator 1a eingesetzten Natriumcarbonat etwa äquimolare Menge an Lithiumcarbonat (Lithiumcarbonat gelöst in destilliertem Wasser, Verhältnis von Nickel : Lithium etwa 1 : 0,07) zugegeben wurde.

Herstellung des Katalysators 1c (Zugabe von Kalium):

Der Katalysator 1b wurde analog zum für den Katalysator 1a beschriebenen Verfahren hergestellt, mit dem Unterschied, dass dabei kein Natriumcarbonat, sondern eine zum für den Katalysator 1a eingesetzten Natriumcarbonat etwa äquimolare Menge an Kaliumcarbonat (Kaliumcarbonat gelöst in destilliertem Wasser, Verhältnis von Nickel: Kalium etwa 1 : 0,07) zugegeben wurde.

Herstellung des Katalysators 1d (keine Zugabe eines Alkali- oder Erdalkalimetalls):

Der Katalysator 1b wurde analog zum für den Katalysator 1a beschriebenen Verfahren hergestellt, mit dem Unterschied, dass dabei kein Natriumcarbonat, also keine Alkali- oder Erdalkalimetallverbindung zugegeben wurde.

Einsatz der Katalysatoren in der Oligomerisierung:

Versuchsreihe 1 (Vergleich von Katalysator 1a und 1d):

In der Versuchsreihe 1 wurden jeweils etwa 4,5 g des Katalysators in einen Differential-Kreislaufreaktor gefüllt und eine Oligomerisierung unter Verwendung des Beschickungsmaterials 1 (siehe Tabelle 1) bei einem Reaktionsdruck von 30 bar und einer Reaktionstemperatur von 100 °C durchgeführt. Für die Versuchreihe 1 wurde eine Belastung von 7,5 g/h an Butenen pro Gramm Katalysator eingesetzt.

Versuchsreiche 2 (Katalysatoren 1a, 1b und 1c):

Es wurden ca. 12 g des Katalysators in ein Metallrohr mit einem Innendurchmesser vom 6 mm eingefüllt. Vor und hinter dem Katalysator wurden Glasperlen mit einem Durchmesser von 2 mm gegeben, die als Vorheiz- bzw. Abkühlphase dienen. Die Oligomerisierung wurde unter Verwendung von zwei unterschiedlichen Beschickungsströmen 2 und 3 bei 30 bar und einer Belastung von 7,5 g/h Buten pro Gram Katalysator durchgeführt, wobei die Reaktionstemperatur 100 °C betrug.

Tabelle 1: Zusammensetzung der Beschickungsströme

|  | Beschickungsstrom 1 | Beschickungsstrom 2 | Beschickungsstrom 3 |
|---|---|---|---|
| iso-Butan | 0,1 % | 8,0 % | 0,3 % |
| n-Butan | 47,5 % | 15,3 % | 78,9 % |
| trans-2-Buten | 15,5 % | 27,9 % | 13,2 % |

(fortgesetzt)

|  | Beschickungsstrom 1 | Beschickungsstrom 2 | Beschickungsstrom 3 |
|---|---|---|---|
| 1-Buten | 28,5 % | 32,7 % | 1,2 % |
| iso-Buten | 0,1 % | 0,9 % | 0,1 % |
| cis-2-Buten | 8,3 % | 15,2 % | 6,1 % |

Die für die jeweiligen Beschickungsströme in den Versuchsreihen 1 und 2 in Abhängigkeit von der Temperatur erzielten Umsätze und Selektivitäten, sowie die daraus resultierenden ISO-Indices sind in den Tabellen 2, 3 und 4 angegeben.

Tabelle 2: Ergebnisse der Oligomerisierung in Versuchsreihe 1:

|  | Katalysator 1a (erfindungsgemäß) | Katalysator 1d (nicht erfindungsgemäß) |
|---|---|---|
| Umsatz n-Buten | 12,2 % | 17,3 % |
| 3,4-DMH* | 11,9 % | 20,0 % |
| 3-MH* | 58,6 % | 58,9 % |
| n-O* | 29,2 % | 19,6 % |
| ISO-Index | 0,79 | 0,97 |

Tabelle 3: Ergebnisse der Oligomerisierung in Versuchsreihe 2 mit Beschickungsstrom 2

| Belastung (Zulauf C4-Olefine in g/h pro Masse Katalystor in g) als WSHV : 7,5 h$^{-1}$ | | | |
|---|---|---|---|
|  | Temperatur | Umsatz | ISO-Index |
|  |  | bezogen auf C4-Olefine |  |
| Katalysator 1a | 100 °C | 21,5 | 0,97 |
| Katalysator 1b | 100 °C | 29,9 | 0,97 |
| Katalysator 1c | 100 °C | 20,8 | 0,95 |

Tabelle 4: Ergebnisse der Oligomerisierung in Versuchsreihe 2 mit Beschickungsstrom 3

| Belastung (Zulauf C4-Olefine in g/h pro Masse Katalystor in g) als WSHV : 7,5 h$^{-1}$ | | | |
|---|---|---|---|
|  | Temperatur | Umsatz | ISO-Index |
|  |  | bezogen auf C4-Olefine |  |
| Katalysator 1a | 100 °C | 17,6 | 1,08 |
| Katalysator 1b | 100 °C | 21,1 | 1,08 |
| Katalysator 1c | 100 °C | 15,8 | 1,06 |
| 3,4-DMH = 3,4-Dimethylhexen 3-MH = 3-Methylhepten n-O = n-Okten | | | |

Zusammenfassend zeigen die vorliegenden Ergebnisse in Versuchsreihe 1, dass durch Zugabe von einer Alkalimetallverbindung wie Natriumcarbonat während der Herstellung des Katalysators der Umsatz zwar reduziert wird, der Anteil an linearen Okten-Isomeren jedoch signifikant erhöht wird. Da jedes Molekül nur einmal umgesetzt werden kann, ist die Bildung der linearer Produkte einem hohen Umsatz vorzuziehen.

Die Ergebnisse der Versuchsreihe 2 zeigen, dass alle getesteten Alkalimetallverbindungen gute Umsätze und hohe Selektivitäten zu linearen Oligomerisierungsprodukten (ISO-Index < 1,1) zeigen. Im Hinblick auf die Bildung von n-Oktenen liefern alle Katalysatoren vergleichbare Ergebnisse, sodass ganz allgemein davon ausgegangen werden kann, dass durch die Zugabe von verschiedenen Alkalimetallverbindungen eine Verbesserung gegenüber Katalysatoren ohne zusätzliche Alkalimetallverbindung erreicht werden kann.

**Patentansprüche**

1. Oligomerisierungskatalysator, welcher Nickeloxid, Al-haltigen und Si-freien Binder (<0,1 Gew.-% Si), ein Silica-Alumina-Supportmaterial und ein Alkali- oder Erdalkalimetalloxid umfasst, wobei der Katalysator eine Zusammensetzung von 15 bis 40 Gew.-%, vorzugsweise 15 bis 30 Gew.-% NiO, 10 bis 30 Gew.-%, vorzugsweise 12 bis 30 Gew.-% $Al_2O_3$, 55 bis 70 Gew.-% $SiO_2$ und 0,01 bis 2,5 Gew.-%, vorzugsweise 0,05 bis 2 Gew.-% eines Alkalimetall- oder Erdalkalimetalloxids aufweist und wobei der Oligomerisierungskatalysator sich durch ein molares Verhältnis Nickelionen : Alkali-/Erdalkalionen im Bereich von 1 : 0,1 bis 1 : 0,001 auszeichnet und im Wesentlichen frei von Titandioxid und Zirkoniumdioxid ist.

2. Oligomerisierungskatalysator nach Anspruch 1, welcher ein molares Verhältnis Nickelionen : Aluminiumionen : Alkali-/Erdalkaliionen im Bereich von 1 : 2 : 0,1 bis 1 : 0,01 : 0,001 aufweist.

3. Oligomerisierungskatalysator nach Anspruch 1 oder 2, wobei als Alkalimetalloxid oder Erdalkalimetalloxid ein Oxid von Lithium, Natrium, Kalium, Magnesium, Calcium oder eine Mischung daraus vorliegt.

4. Oligomerisierungskatalysator nach einem der Ansprüche 1 bis 3, wobei der Oligomerisierungskatalysator eine spezifische BET-Oberfläche von 150 bis 400 $m^2/g$, bestimmt mittels Stickstoff-Physisorption, aufweist.

5. Verfahren zur Herstellung des Oligomerisierungskatalysators nach einem der Ansprüche 1 bis 4, umfassend mindestens die Schritte:

   a) Mischen des amorphen Silica-Alumina-Supportmaterials, des Al-haltigen und Si-freien Binders (<0,1 Gew.-% Si),mindestens eines Teils der Nickelquelle und optional der Alkali- oder Erdalkaliquelle; und Granulieren der so hergestellten Mischung;
   a1) Beaufschlagen des in Schritt a) hergestellten Granulats mit mindestens einem Teil einer Nickelquelle und/oder einer einer Alkali- oder Erdalkaliquelle, sofern die gesamte Nickelquelle und/oder die Alkalimetall- oder Erdalkalimetallquelle nicht schon in Schritt a) mit dem amorphen Silica-Alumina-Supportmaterial und dem Al-haltigen und Si-freien Binder vermischt worden ist,
   wobei der Anteil des amorphen Silica-Alumina-Supportmaterials an dem Gesamtansatz (Gesamtzusammensetzung inklusive aller ggf. verwendeten Lösemittel) nach Schritt a) oder a1) 20 bis 50 Gew.-%, der Anteil des Al-haltigen und Si-freien Binders an dem Gesamtansatz 5 bis 30 Gew.-%, der Anteil der Alkali- oder Erdalkaliquelle in nicht gelöster Form an dem Gesamtansatz 0,01 und 2,5 Gew.-% und der Anteil der Nickelquelle an dem Gesamtansatz 30 und 50 Gew.-%, beträgt; und
   b) Trocknen und Kalzinieren des Granulats zur Herstellung des Oligomerisierungskatalysators.

6. Verfahren nach Anspruch 5, wobei als Al-haltiger und Si-freier Binder in Schritt a) ein oxidisches Aluminiummaterial, vorzugsweise Aluminiumoxid, Aluminiumhydroxid oder Aluminiumoxid-hydroxid, eingesetzt wird.

7. Verfahren nach Anspruch 5 oder 6, wobei als amorphes Silica-Alumina-Supportmaterial ein amorphes Alumosilicat eingesetzt wird.

8. Verfahren nach einem der Ansprüche 5 bis 7, wobei die Kalzination in Schritt b) bei einer Temperatur zwischen 400 °C und 800 °C durchgeführt wird.

9. Verfahren nach einem der Ansprüche 5 bis 8, wobei der Oligomerisierungskatalysator eine finale Zusammensetzung von 15 bis 40 Gew.-%, vorzugsweise 15 bis 30 Gew.-% NiO, 10 bis 30 Gew.-% $Al_2O_3$, 55 bis 70 Gew.-% $SiO_2$ und 0,01 bis 2,5 Gew.-%, vorzugsweise 0,05 bis 2 Gew.-% eines Alkalimetall- oder Erdalkalimetalloxids aufweist.

10. Verfahren zur Oligomerisierung von C3- bis C6-Olefinen, wobei ein olefinhaltiges Einsatzgemisch, welches die C3- bis C6-Olefine enthält, in einer Reaktionszone mit einem Oligomerisierungskatalysator in Kontakt gebracht wird, wobei als Oligomerisierungskatalysator der Katalysator nach einem der Ansprüche 1 bis 4 eingesetzt wird.

**11.** Verfahren zur Oligomerisierung nach Anspruch 10, wobei C3- bis C5-Olefine oligomerisiert werden und das olefinhaltige Einsatzgemisch die C3- bis C5-Olefine enthält.

**12.** Verfahren zur Oligomerisierung nach Anspruch 10, wobei C4-Olefine oligomerisiert werden und das olefinhaltige Einsatzgemisch die C4-Olefine enthält.

**13.** Verfahren zur Oligomerisierung nach einem der Ansprüche 10 bis 12, wobei das olefinhaltige Einsatzgemisch weniger als 2 Gew.-% verzweigte Olefine enthält

**14.** Verfahren zur Oligomerisierung nach einem der Ansprüche 10 bis 13, wobei die Oligomerisierung in flüssiger Phase stattfindet.

**15.** Verfahren zur Oligomerisierung nach einem der Ansprüche 10 bis 14, wobei die Oligomerisierung bei einem Druck von 10 bis 70 bar und einer Temperatur von 50 bis 200 °C durchgeführt wird, unter der Voraussetzung, dass, sofern die Oligomerisierung in flüssiger Phase durchgeführt wird, die Parameter Druck und Temperatur so gewählt werden, dass der Eduktstrom in flüssiger Phase vorliegt.

Europäisches Patentamt

European Patent Office

Office européen des brevets

## EUROPÄISCHER RECHERCHENBERICHT

Nummer der Anmeldung

EP 19 16 2076

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 3 557 242 A (SAMPSON ROY JOHN ET AL) 19. Januar 1971 (1971-01-19) | 1-4, 10-15 | INV. B01J21/12 |
| Y | * Ansprüche 1-3; Beispiele 4,6(B)(ii) * ----- | 5-9 | B01J23/02 B01J23/755 |
| Y | US 3 658 935 A (PINE LLOYD A) 25. April 1972 (1972-04-25) * Spalte 4, Zeilen 18-23; Anspruch 1 * ----- | 1-15 | B01J35/00 B01J37/00 C07C2/10 |
| Y | US 2 581 228 A (BAILEY GRANT C ET AL) 1. Januar 1952 (1952-01-01) * Spalte 9, Zeilen 22-24 * * Spalte 6, Zeilen 68-74 * * Beispiele 4-6 * ----- | 1-15 | |
| Y | WO 2010/117539 A2 (UOP LLC [US]; NICHOLAS CHRISTOPHER P [US]; BHATTACHARYYA ALAKANANDA [U) 14. Oktober 2010 (2010-10-14) * Absätze [0032], [0040]; Abbildung 2; Beispiele 2,3,7,8 * ----- | 1-15 | |
| Y | DE 10 2009 027408 A1 (EVONIK OXENO GMBH [DE]) 5. Januar 2011 (2011-01-05) * Absätze [0003], [0037]; Ansprüche 7,8; Beispiele 1,2 * ----- | 1-15 | RECHERCHIERTE SACHGEBIETE (IPC) B01J C07C |
| Y | WO 93/06926 A1 (ENIRICERCHE SPA [IT]) 15. April 1993 (1993-04-15) * Seite 5, Zeile 24 - Seite 6, Zeile 10; Beispiel 1 * ----- | 1-15 | |
| Y | WO 95/14647 A1 (BASF AG [DE]; VICARI MAXIMILIAN [DE]; POLANEK PETER [DE]) 1. Juni 1995 (1995-06-01) * Seite 5, Zeilen 23-28 * * Seite 6, Zeilen 28-33; Beispiele 1, Vergleichsbeispiel 1 * ----- -/-- | 1-6,8, 10-15 | |

Der vorliegende Recherchenbericht wurde für alle Patentansprüche erstellt

| Recherchenort | Abschlußdatum der Recherche | Prüfer |
|---|---|---|
| München | 23. August 2019 | Goebel, Matthias |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

                          

& : Mitglied der gleichen Patentfamilie, übereinstimmendes Dokument

EPO FORM 1503 03.82 (P04C03)

Seite 1 von 2

Europäisches
Patentamt

European
Patent Office

Office européen
des brevets

# EUROPÄISCHER RECHERCHENBERICHT

**Nummer der Anmeldung**

EP 19 16 2076

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | MOUSSA SARA ET AL: "Heterogeneous oligomerization of ethylene to liquids on bifunctional Ni-based catalysts: The influence of support properties on nickel speciation and catalytic performance", CATALYSIS TODAY, ELSEVIER, AMSTERDAM, NL, Bd. 277, 11. Januar 2016 (2016-01-11), Seiten 78-88, XP029743467, ISSN: 0920-5861, DOI: 10.1016/J.CATTOD.2015.11.032 * Seite 80, Absatz 2 * * Seite 81, Absatz 4 * * Seite 86, linke Spalte * ----- | 1-15 | |

**RECHERCHIERTE SACHGEBIETE (IPC)**

Der vorliegende Recherchenbericht wurde für alle Patentansprüche erstellt

| Recherchenort | Abschlußdatum der Recherche | Prüfer |
|---|---|---|
| München | 23. August 2019 | Goebel, Matthias |

KATEGORIE DER GENANNTEN DOKUMENTE

X : von besonderer Bedeutung allein betrachtet
Y : von besonderer Bedeutung in Verbindung mit einer
  anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
  nach dem Anmeldedatum veröffentlicht worden ist
D : in der Anmeldung angeführtes Dokument
L : aus anderen Gründen angeführtes Dokument

  ........................................................................................
& : Mitglied der gleichen Patentfamilie, übereinstimmendes
  Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 19 16 2076

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

23-08-2019

| Im Recherchenbericht angeführtes Patentdokument | | Datum der Veröffentlichung | Mitglied(er) der Patentfamilie | | Datum der Veröffentlichung |
|---|---|---|---|---|---|
| US 3557242 | A | 19-01-1971 | BE | 729350 A | 04-09-1969 |
| | | | DE | 1911030 A1 | 25-09-1969 |
| | | | FR | 2003215 A1 | 07-11-1969 |
| | | | GB | 1195307 A | 17-06-1970 |
| | | | NL | 6903344 A | 08-09-1969 |
| | | | US | 3557242 A | 19-01-1971 |
| US 3658935 | A | 25-04-1972 | KEINE | | |
| US 2581228 | A | 01-01-1952 | GB | 619231 A | 07-03-1949 |
| | | | US | 2581228 A | 01-01-1952 |
| WO 2010117539 | A2 | 14-10-2010 | BR | PI1013710 A2 | 05-04-2016 |
| | | | CN | 102448913 A | 09-05-2012 |
| | | | CN | 104276911 A | 14-01-2015 |
| | | | EP | 2414310 A2 | 08-02-2012 |
| | | | JP | 5553889 B2 | 16-07-2014 |
| | | | JP | 2012522109 A | 20-09-2012 |
| | | | KR | 20120001795 A | 04-01-2012 |
| | | | TW | 201040251 A | 16-11-2010 |
| | | | WO | 2010117539 A2 | 14-10-2010 |
| DE 102009027408 | A1 | 05-01-2011 | DE | 102009027408 A1 | 05-01-2011 |
| | | | WO | 2011000697 A1 | 06-01-2011 |
| WO 9306926 | A1 | 15-04-1993 | AT | 133583 T | 15-02-1996 |
| | | | DE | 69208095 D1 | 14-03-1996 |
| | | | DE | 69208095 T2 | 22-08-1996 |
| | | | DK | 0606314 T3 | 09-04-1996 |
| | | | EP | 0606314 A1 | 20-07-1994 |
| | | | ES | 2083193 T3 | 01-04-1996 |
| | | | GR | 3019107 T3 | 31-05-1996 |
| | | | IT | 1251614 B | 17-05-1995 |
| | | | RU | 2094119 C1 | 27-10-1997 |
| | | | US | 5510555 A | 23-04-1996 |
| | | | WO | 9306926 A1 | 15-04-1993 |
| WO 9514647 | A1 | 01-06-1995 | DE | 4339713 A1 | 24-05-1995 |
| | | | EP | 0730567 A1 | 11-09-1996 |
| | | | ES | 2122510 T3 | 16-12-1998 |
| | | | JP | 3544980 B2 | 21-07-2004 |
| | | | JP | H09505618 A | 03-06-1997 |
| | | | US | 5849972 A | 15-12-1998 |
| | | | WO | 9514647 A1 | 01-06-1995 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

**In der Beschreibung aufgeführte Patentdokumente**

- WO 9514647 A1